(19) Europäisches Patentamt
European Patent Office
Office européen des brevets

(11) Veröffentlichungsnummer: **0 046 856**
**A2**

(12) # EUROPÄISCHE PATENTANMELDUNG

(21) Anmeldenummer: 81105426.1

(22) Anmeldetag: 11.07.81

(51) Int. Cl.³: C 07 D 239/47

(30) Priorität: 29.08.80 DE 3032548
29.11.80 DE 3045076

(43) Veröffentlichungstag der Anmeldung:
10.03.82 Patentblatt 82/10

(84) Benannte Vertragsstaaten:
AT BE CH DE FR GB IT LI LU NL SE

(71) Anmelder: DYNAMIT NOBEL AKTIENGESELLSCHAFT
Patentabteilung Postfach 1209
D-5210 Troisdorf, Bez. Köln(DE)

(72) Erfinder: Peeters, Hermann, Dr.
Porzer Strasse 1
D-5216 Niederkassel-Ranzel(DE)

(72) Erfinder: Vogt, Wilhelm, Dr.
Neuenhöfer Allee 53
D-5000 Köln 41(DE)

(54) **Verfahren zur Herstellung von 4-Amino-2-mercaptopyrimidinen.**

(57) 4-Amino-2-mercaptopyrimidine, die in 5-Stellung substituiert sein können, werden aus 3-Alkoxyacrylnitrilen, deren Acetalen oder 3-Aminoacrylnitrilen, die entsprechend substituiert sein können, durch Umsetzung mit Thioharnstoff hergestellt.

EP 0 046 856 A2

Croydon Printing Company Ltd.

Troisdorf, den 21. Mai 1981
OZ: 80048/ 80 087 Ausland

DYNAMIT NOBEL AKTIENGESELLSCHAFT
Troisdorf, Bez. Köln

Verfahren zur Herstellung von 4-Amino-2-mercapto-pyrimidinen

Die Erfindung betrifft ein Verfahren zur Herstellung von 4-Amino-2-mercaptopyrimidinen.

Zur Herstellung von 4-Amino-2-mercaptopyrimidinen der Formel (1) ist die Umsetzung von Uracil, das gegebenenfalls in 5-Stellung auch substituiert sein kann, mit Phosphorpentasulfid und anschließende Substitution der 4-Mercaptogruppe im so erhaltenen 2,4-Dimercaptopyrimidin mit Hilfe von Ammoniak ( US-PS 2 682 542 ), oder die Kondensation von Cyanessigester mit Thioharnstoff zum 4-Amino-5-carbethoxy-2-mercaptopyrimidin und anschließende Verseifung und Decarboxylierung ( J.org.Chem. 27 (1962) 4211 ), Hydrolyse des nur in einem mehrstufigen Verfahren erhältlichen 4-Amino-2-methylmercaptopyrimidin mit Natrium/Ammoniak (Bull.Soc.Chim.Belges 79 (1970) 329) und die Umsetzung von 2-Thiouracil mit Phosphorsäuretriamid (Isv. Akad. SSSR 1969, 655 ) beschrieben.

Die nach dem Stand der Technik bekannten Verfahren weisen alle große Nachteile auf durch Verwendung teurer Reagentien und/oder aufwendige Verfahrensschritte.

Aufgabe der vorliegenden Erfindung war die Schaffung eines Verfahrens zur Herstellung von 4-Amino-2-mercaptopyrimidinen bei einfacher Reaktionsführung und Verwendung preiswerter Ausgangs- und Hilfsstoffe.

Diese Aufgabe wurde durch eine Umsetzung nach folgender Gleichung gelöst

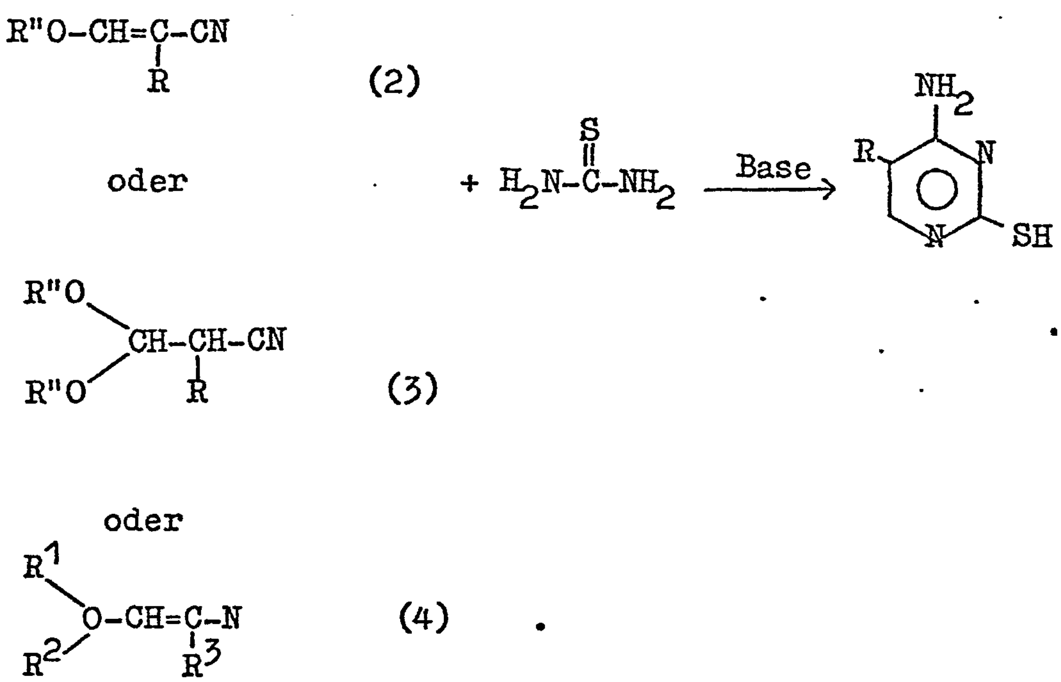


(2)

oder

(3)

oder

(4) .

Gegenstand der Erfindung ist ein Verfahren zur Herstellung von 4-Amino-2-mercaptopyrimidinen der Formel

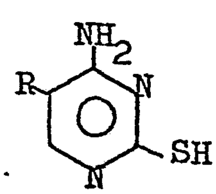

(1)

Methylglykol oder polar-aprotischen Lösungsmitteln wie z.B. Dimethylformamid oder Dimethylsulfoxid gearbeitet wird.

3. Verfahren nach Anspruch 1, dadurch gekennzeichnet, daß 3-Aminoacrylnitril in Anwesenheit fremder Lösungsmittel mit einem Überschuß von 3-Aminoacrylnitril als Lösungsmittel umgesetzt wird.

4. Verfahren nach mindestens einem der Ansprüche 1 bis 3, dadurch gekennzeichnet, daß in Gegenwart von Alkali- oder Erdalkalihydroxiden und Alkali- und Erdalkalialkoholaten als Basen gearbeitet wird.

5. Verfahren nach mindestens einem der Ansprüche 1 bis 4, dadurch gekennzeichnet, daß das Molverhältnis von Alkoxyacrylnitril (2) bzw. Acetal (3) zu Base 1 zu 1,0 bis 1 zu 3,0, vorzugsweise 1 zu 1,0 bis 1 zu 1,5 beträgt.

6. Verfahren nach mindestens einem der Ansprüche 1 bis 4, dadurch gekennzeichnet, daß bei der Umsetzung von 3-Aminoacrylnitril das Molverhältnis Thioharnstoff zu Base 0,5 zu 1 bis 1,5 zu 1 beträgt.

7. Verfahren nach mindestens einem der Ansprüche 1 bis 5, dadurch gekennzeichnet, daß das Molverhältnis von Alkoxyacrylnitril (2) bzw. Acetal (3) zur Thioharnstoff 1 zu 1,0 bis 1 zu 2,0, vorzugsweise 1 zu 1,0 bis 1 zu 1,5 beträgt.

8. Verfahren nach mindestens einem der Ansprüche 1 bis 4, dadurch gekennzeichnet, daß das Molverhältnis 3-Aminoacrylnitril zu Thioharnstoff 1 zu 1 bis 4 oder mehr zu 1 beträgt.

9. Verfahren nach mindestens einem der Ansprüche 1 bis 8, dadurch gekennzeichnet, daß die Umsetzung im Temperaturbereich von 40 bis 250°C erfolgt.

10. Verfahren nach mindestens einem der Ansprüche 1 bis 9, dadurch gekennzeichnet, daß der Druck zwischen Normaldruck und 30 bar liegt.

Dr.La/Ra

din, hochsiedende Amine oder Dimethylsulfoxid, oder Gemische von unpolaren Lösungsmitteln wie polaren Lösungsmitteln ( z.B. Toluol/Alkohol-Gemische) in Frage. Ein weiteres geeignetes Lösungsmittel ist z.B. Acetonitril. Die Reaktion kann bevorzugt in Gegenwart von Basen oder gegebenenfalls in Abwesenheit von Basen ausgeführt werden.

Dann wird Thioharnstoff und die Base vorteilhaft in Abwesenheit eines fremden Lösungsmittels in dem 3-Aminoacrylnitril, das als Substrat und Lösungsmittel dient, suspendiert und bei erhöhter Temperatur umgesetzt. Das Molverhältnis von Thioharnstoff zu 3-Aminoacrylnitril beträgt im allgemeinen 1 zu 1 bis 1 zu 4, gegebenenfalls kann auch ein größerer Überschuß an 3-Aminoacrylnitril verwendet werden, z.B. um gute Rührbarkeit zu erzielen. In Anwesenheit von Lösungsmitteln kann der Überschuß von 3-Aminoacrylnitril gering sein oder ein geringer Überschuß von Thioharnstoff verwendet werden.

Geeignete Base sind z.B. Alkali- oder Erdalkalihydroxide oder Alkali- oder Erdalkalialkoholate, ebenfalls geeignet sind starke organische Basen wie z.B. tertiäre Amine. Das Molverhältnis von Alkoxyacrylnitril (2) bzw. Acetal (3) zu Base sollte 1 zu 1,0 bis 1 zu 3,0, vorteilhaft 1 zu 1,0 bis 1 zu 1,5 betragen.
Gegebenenfalls können sogenannte Phasentransfer-Katalysatoren, wie quartäre Ammoniumsalze oder quartäre Phosphoniumsalze oder Kronenäther in Mengen von im allgemeinen 0,5 bis 20 Mol %, bezogen auf eingesetzten Thioharnstoff, zur Erhöhung der Ausbeute zugesetzt werden. Phasentransfer-Katalysatoren sind u.a. in Zeitschrift für Angewandte Chemie 89 (1977) 521 beschrieben, auf die Art der quartären Ammoniumsalze und Kronenäther ist näher in der DE-OS 29 12 345 eingegangen.

Das Molverhältnis von Alkoxyacrylnitril (2) bzw. Acetal (3) zu Thioharnstoff sollte 1 zu 1,0 bis 1 zu 2,0 ,vorteilhaft 1 zu 1,0 bis 1 zu 1,5 betragen.

Die Konzentration der Reaktionsteilnehmer richtet sich hauptsächlich nach ihrer Löslichkeit, im allgemeinen liegt die Anfangskonzentration des Alkoxyacrylnitrils (2) bzw. des Acetals (3) bei 0,5 bis 10 mol/l, vorteilhaft bei 1 bis 4 mol/l.

Die Umsetzung erfolgt bei erhöhter Temperatur im Bereich von 40 bis 250 °C, vorteilhaft bei der Rückflußtemperatur des Lösungsmittels. Zur Umsetzung der Stoffe der Formeln (2) und (3) sind 40 bis 160°C, der Stoffe der Formel (4) 100 bis 200°C bevorzugte Temperaturen.
Die Reaktion erfolgt im allgemeinen unter Normaldruck, doch ist gegebenenfalls auch ein Arbeiten unter Überdruck, beispielsweise bis 30 bar, möglich.

Die Reaktionszeit beträgt etwa 0,2 bis 10 h, im allgemeinen 0,5 bis 5 h.
Das 4-Amino-2-mercaptopyrimidin der Formel (1) fällt meistens zunächst als wasserlösliches Alkali- oder Erdalkalisalz an, das durch Ansäuern auf einen pH-Wert von 7 ± 3 in die freie, schwer wasserlösliche Verbindung überführt werden kann.

Die Aufarbeitung des Ansatzes erfolgt im allgemeinen so, daß das Lösungsmittel abgezogen, das zurückbleibende Alkali - oder Erdalkalisalz des 4-Amino-2-mercaptopyrimidins in Wasser, dem gegebenenfalls eine anorganische Base zugegeben wird, aufgenommen und die freie Verbindung durch Zugabe einer organischen Säure wie z.B. Essigsäure oder einer Mineralsäure wie Salzsäure oder Schwefelsäure

und Einstellung auf einen pH-Wert von 7 ± 3 isoliert wird, doch kann z.B. auch ohne vorhergehendes Abziehen des Lösungsmittels angesäuert und das Produkt nach Abfiltrieren, Trocknen und gegebenenfalls Umkristallisieren, isoliert werden.

4-Amino-2-mercaptopyrimidine können Verwendung finden z.B. als Zusatz in photographischem Material (Jap.Pat. 52 058532, 50 019428, 48 59827), als Fungizid (NL-Pat. 68 14057) oder als mögliches Chemotherapeutikum: J.cell. Physiol. 78 (1971) 25, (C.A. 76, 87 c); Chromosones Today 1972, 118 (C.A. 81, 100180 Z); Phytopathology 52 (1962) 432 (C.A. 57, 17192 e).

Demgemäß haben die Substituenten R und $R^3$ sich nach der Funktion im jeweiligen Produkt zu richten, so daß R und $R^3$ sehr verschiedenartige Struktur aufweisen können, wobei lediglich ein inertes Verhalten bei der Herstellung die einzige Begrenzung darstellt.

Die Art der substituierten Aminogruppe $R^1R^2N$ - richtet sich im wesentlichen nach der Zugängigkeit des jeweiligen 3-Aminoacrylnitrils, jedoch ebenfalls nach der Trennbarkeit vom Ausgangsstoff und Verunreinigungen vom Produkt u.a.

Besonders genannt sei die Dimethylamino-, die Diäthylamino- und die Morpholinogruppe.

Dr.La/Ra

4-Amino-2-mercaptopyrimidin

Beispiel 1

97 g (1 mol) 3-Aethoxyacrylnitril und 91 g (1,2 mol) Thioharnstoff werden in 460 g einer 16,3 Gew.-%igen äthanolischen Natriumäthylatlösung (1,1 mol Natriumäthylat ) 3 h zum Rückfluß erhitzt. Das Lösungsmittel wird im Vakuum abgezogen und der Rückstand in 900 ml Wasser aufgenommen. Durch Zugabe von Salzsäure wird auf einen pH-Wert von 7 eingestellt und das Produkt ausgefällt. Nach dem Abfiltrieren, Waschen mit Wasser und Trocknen im Vakuum werden 112,3 g (88,4 % d.Th.) 4-Amino-2-mercaptopyrimidin erhalten. Schmp.: 275-80°C (Zers.), Molekülmasse ( massenspektroskopisch ) 127.

Beispiel 2

9,7 g (0,1 mol) 3-Aethoxyacrylnitril und 9,1 g (0,12 mol) Thioharnstoff werden in 100 ml einer Lösung von 0,1 mol n-Butanol in 100 ml n-Butanol 3 h zum Rückfluß erhitzt. Nach Aufarbeitung wie im Beispiel 1 werden 11,2 g (88,2 % d.Th.) Zielprodukt erhalten.

Beispiel 3

9,7 g (0,1 mol) 3-Aethoxyacrylnitril und 9,1 g (0,12 mol) Thioharnstoff werden in 20 g einer 30 proz. methanolischen Natriumlösung (0,11 mol Natriummethylat ) 3 h zum Rückfluß erhitzt. Nach Aufarbeitung wie im Beispiel 1 werden 9,3 g (73,9 % d.Th.) Zielprodukt erhalten.

Beispiel 4

9,7 g (0,1 mol) 3-Aethoxyacrylnitril und 9,1 g (0,12 mol) Thioharnstoff werden in 82,5 g einer 8 proz. äthanolischen Natriumäthylatlösung (0,11 mol Natriumäthylat) 3 h zum Rückfluß erhitzt. Nach Aufarbeitung wie im Beispiel 1 werden 9,1 g (71,7 % d.Th.) Zielprodukt erhalten.

Beispiel 5

14,6 g (0,1 mol) Cyanacetaldehyddiäthylacetal und 9,1 g (0,12 mol) Thioharnstoff werden in 46 g einer 16,3 proz. äthanolischen Natriumäthylatlösung (0,11 mol Natriumäthylat ) 3 h zum Rückfluß erhitzt. Nach Aufarbeitung wie im Beispiel 1 werden 11,2 g (88,2 % d.Th.) Zielprodukt erhalten.

4-Amino-2-mercapto-5-methylpyrimidin

Beispiel 6

8,3 g (0,075 mol) 3-Aethoxy-2-methylacrylnitril und 6,8 g (0,09 mol) Thioharnstoff werden in 34,5 g einer 16,3 proz. äthanolischen Natriumäthylatlösung (0,085 mol Natriumäthylat) 3 Stunden zum Rückfluß erhitzt. Nach Aufarbeitung wie im Beispiel 1 werden 6,5 g (61,5 % d.Th.) 4-Amino-2-mercapto-5-methylpyrimidin erhalten. Schmp.: 265-70°C ( Zers.) Molekülmasse (massenspektroskopisch) 141.

4-Amino-2-mercapto-5-benzylpyrimidin

Beispiel 7

14 g (0,075 mol) 3-Aethoxy-2-benzylacrylnitril und 6,8 g (0,09 mol) Thioharnstoff werden in 34,5 g einer 16,3 proz. äthanolischen Natriumäthylatlösung (0,0825 mol Natriumäthylat) 3 h zum Rückfluß erhitzt. Nach Aufarbeitung wie im Beispiel 1 werden 15,3 g (94,0 % d.Th.) 4-Amino-5-benzyl-2-mercaptopyrimidin erhalten. Schm.: 295-305°C (Zers.), Molekülmasse ( massenspektroskopisch) 217.

4-Amino-2-mercapto-5-methoxymethylpyrimidin

Beispiel 8

7,1 g (0,05 mol) 3-Aethoxy-2-methoxymethylacrylnitril und 4,6 g (0,06 mol) Thioharnstoff werden in 20,5 g einer 1,83 % äthanolischen Natriumäthylatlösung (0,055 mol Natriumäthylat) 3 h zum Rückfluß erhitzt. Nach Aufarbeitung wie im Beispiel 1 werden 6,8 g (79,5 % d.Th.) 4-Amino-2-mercapto-5-methoxymethylpyrimidin erhalten Schmp. 260-70°C (Zers.) Molekülmasse (massenspektroskopisch) 171

4-Amino-2-mercaptopyrimidin

Beispiel 9

5,7 g (0,075 mol) Thioharnstoff, 19,2 g (0,2 mol) 3-Dimethylaminoacrylnitril und 4,05 g (0,075 mol) Natriummethylat (fest) wurden unter $N_2$ 2 Stunden auf 140°C erhitzt. Nach Zugabe von 50 ml 10 proz. Natronlauge entstanden zwei Phasen. Die organische Phase wurde nach Zugabe von Methylenchlorid abgetrennt, getrocknet und vom Lösungsmittel befreit.
Rückstand 11,2 g 3-Dimethylaminoacrylnitril.

Die wässrige Phase wurde mit konzentrierter Salzsäure auf einen pH-Wert von 7 gebracht. Dabei fiel ein Feststoff aus, der abfiltriert und getrocknet wurde: 6,86 g (72,0 %) 4-Amino-2-mercaptopyrimidin. Schmp.: 272-4°C.

Beispiel 10

3,8 g (0,05 mol) Thioharnstoff, 14,4 g (0,15 mol) 3-Dimethylamonoacrylnitril und 3,74 g (0,055 mol) Natriumethylat (fest) wurden wie im Beispiel 9 umgesetzt und aufgearbeitet.

Rückstand: 9,4 g 3-Dimethylaminoacrylnitril. 4-Amino-2-mercaptopyrimidin: 1,8 g (28,3 % d.Th.)

Beispiel 11

Beispiel 10 wird wiederholt, aber unter Zusatz von 1,61 g (0,005 mol) Tetra-n-butylammoniumbromid.
Rückstand: 11,6 g 3-Dimethylaminoacrylnitril. 4-Amino-2-mercaptopyrimidin: 2,67 g (42,0 % d.Th.)

Beispiel 12

3,04 g (0,04 mol) Thioharnstoff, 2,7 g (0,05 mol) Natriummethylat und 13,8 g 3-Morpholinoacrylnitril werden 3 Stunden unter $N_2$ auf 140°C erhitzt. Nach Aufarbeitung wie im Beispiel 9 wurden 6,3 g (45,7 % d.Th.) 3-Morpholinoacrylnitril zurückgewonnen und 1,95 g (38,4 %) 4-Amino-2-mercaptopyrimidin erhalten.

4-Amino-2-mercapto-5-methylpyrimidin
Beispiel 13

1,9 g (0,025 mol) Thioharnstoff, 1,49 g (0,027 mol) Natriummethylat und 5,5 g (0,05 mol) 3-Dimethylamino-2-methylacrylnitril werden 5 Stunden unter $N_2$ auf 140°C erwärmt. Nach Aufarbeitung wie im Beispiel 9 wurden 0,84 g (23,9 % d.Th.) 4-Amino-2-mercapto-5-methylpyrimidin erhalten. Schmp.: 263-7°C

4-Amino-2-mercapto-5-benzylpyrimidin

Beispiel 14

3,8 g (0,05 mol) Thioharnstoff, 2,96 g (0,055 mol) Natriummethylat und 18,6 g (0,1 mol) 3-Dimethylamino-2-benzylacrylnitril werden 5 Stunden unter $N_2$ auf 140°C erhitzt. Nach Aufarbeitung wie im Beispiel 9 wurden 5,6 g (51,6 % d.Th.) 4-Amino-2-mercapto-5-benzylpyrimidin erhalten. Schmp.: 300-302°C (Zers.) .

Dr.La/Ra

## Patentansprüche

1. Verfahren zur Herstellung von 4-Amino-2-mercaptopyrimidinen der Formel

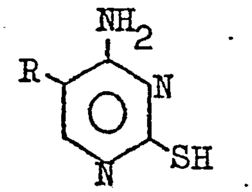

(1)

worin R ein Wasserstoff, geradkettige, verzweigte oder cyclische Alkylreste mit 1 bis 20 Kohlenstoffatomen, geradkettige oder verzweigte Reste $(CH_2)_n-CN$, $(CH_2)_nCOOR'$, $(CH_2)_nNH_2$, $(CH_2)_nOR'$, $(CH_2)_nCyc$, wobei Cyc ein isocyclischer oder heterocyclischer Ring mit ein- oder mehrcyclischer Struktur oder ein aromatischer oder heteroaromatischer Ring mit ein- oder mehrcyclischer Struktur, der gegebenenfalls Substituenten am Ring tragen kann, R' Alkylreste mit 1 bis 12 Kohlenstoffatomen oder Reste einwertiger Phenole und n = 1 bis 5 ist, bedeutet, dadurch gekennzeichnet, daß ein 3-Alkoxyacrylnitril der Formel

$$R''O-CH=\underset{R}{\underset{|}{C}}-CN \qquad (2)$$

worin R die obengenannte Bedeutung hat und R" die Bedeutung geradkettige oder verzweigte Alkyl- oder Alkenylreste mit 1 bis 12 Kohlenstoffatomen, isocyclische oder heterocyclische, einkernige oder mehrkernige aromatische oder cycloaliphatische Ringsysteme, welche gegebenenfalls Substituenten tragen, oder $(CH_2)_mCyc$ mit Cyc in der vorigen Bedeutung, die Reste $(CH_2)_mOR'''$ oder $(CH_2-CH_2-O)_pR'''$ mit m = 1 - 5 und p = 1 bis 4 und R"' geradkettige oder verzweigte Alkylreste mit 1 bis 12 Kohlenstoffatomen hat, oder ein Acetal

der Formel

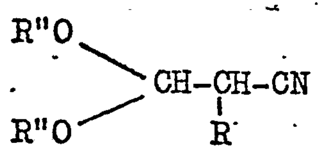

(3)

wobei R und R" die obengenannte Bedeutung haben oder ein 3-Aminoacrylnitril der Formel

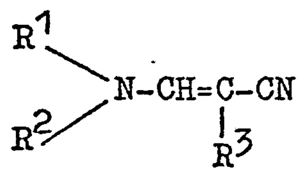

(4)

worin $R^1$ und $R^2$ die Bedeutung H, gleiche oder verschiedene, geradkettige oder verzweigte Alkyl-, Alkenyl- oder Alkinyl-Reste mit 1 bis 12 C-Atomen, -Cyc, $-(CH_2)_n$-Cyc, worin Cyc isocyclische oder heterocyclische, einkernige oder mehrkernige, aromatische oder cycloaliphatische Ringsysteme, welche gegebenenfalls Substituenten an den Ringen tragen können und n = 0-5 ist, die Gruppierung X-$R^4$, worin X geradkettig, verzweigt oder cyclische Alkylen- oder Alkenyl-Reste mit 2 bis 12 C-Atomen, -Cyc, $-(CH_2)_n$-Cyc- oder $-(CH_2)_n$-Cyc-$(CH_2)_n$- mit Cyc und n in der vorigen Bedeutung und $R^4$ =

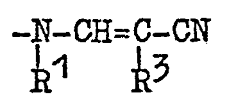

,

oder $R^1$ und $R^2$ zusammen Alkylen- oder Alkenylen-Reste eines Ringes mit 3 bis 6 Gliedern bilden, der gegebenenfalls durch ein oder mehrere Heteroatome unterbrochen ist, und $R^3$ die genannte Bedeutung hat, mit Thioharnstoff umgesetzt wird.

2. Verfahren nach Anspruch 1, dadurch gekennzeichnet, daß in polaren Lösungsmitteln oder in Gegenwart polarer Lösungsmittel wie Wasser, Alkoholen, bevorzugt solchen mit 1 bis 4 Kohlenstoffatomen, Aetheralkoholen wie